# EUROPEAN PATENT SPECIFICATION

(11) **EP 2 140 852 B1**
(45) Date of publication and mention of the grant of the patent: **24.10.2012**
(21) Application number: 08011781.5
(22) Date of filing: 30.06.2008
(51) Int. Cl.: A61K 8/06, A61Q 1/12

(54) **Foundation with high chromatic stability and method for obtaining it**
Grundierung mit hoher chromatischer Stabilität und Verfahren zu ihrer Herstellung
Fond de teint à forte stabilité chromatique et son procédé d'obtention

(43) Date of publication of application: 06.01.2010
(73) Proprietor: Prodotti Gianni S.p.A., 20138 Milano (IT)
(72) Inventor: Gianni, Stefano, 20138 Milano (IT)
(74) Representative: Ferreccio, Rinaldo

(56) References cited:
- US-A- 4 119 712
- US-B1- 6 284 228

## Description

### Prior art

Foundation is a cosmetic composition applicable on the face and other parts of the body, useful for giving skin a pre-determined colour and/or covering possible blemishes. Foundations are generally emulsified creams, containing different pigments, duly mixed, so to reproduce the natural colour of the skin. Their related compositions contain lipophilic phases, for example silicones, emulsified with water by means of special surfactants.

In this field, it is particularly important to obtain compositions in a wide range of colours, whose chromatic characteristics can be easily controlled and reproduced, and having high stability characteristics in emulsified form. Typically, in the production of foundations, it is required to obtain a very high number of compositions in a very limited chromatic range, in order to reproduce the numerous natural nuances of the human skin in the most faithful manner. Within this limited chromatic range, colour differences between one composition and another are very small: hence, it is necessary to obtain highly stable compositions, reproducible as for final colour.

Normally, the preparation of the foundation envisages the creation of a primary emulsion, to which the mixture of pigments required for obtaining the desired colour tone is added; in other cases, pigments can be emulsified directly with the other ingredients, obtaining the final foundation, in a single step; in all these techniques, the final colour of the foundation is determined on the basis of qualitative-quantitative formulations of different pigments, which are used as such (i.e. as they are available on the market, for example in powder form) together with the other ingredients of the foundation. These techniques have some limits, especially with respect to the definition of the final colour obtained, which is not always optimal. In fact, it has been noticed that, starting from identical pigment formulations and subjecting them to the same formulation process, foundations were obtained with significant colour variations among different batches or even within one single production batch.

Recently, in US-A-6,284,228, it has been proposed to create a foundation by mixing different, separately prepared components, respectively: one light component, one red component, one green component and one optional dark component; the colour of the foundation results from mixing of the abovementioned components; in turn, each component contains mixtures of different pigments, to be mixed with one another as such, in specific percentages: therefore, this technique shows the same limits of the original technique, i.e. the variability of the final colour, since it is predetermined starting from mixtures of pigments as such.

Notwithstanding the wide research carried out in this sector, at the moment, the requirement of foundation compositions with high chromatic stability is not yet met; in addition, there is still a need for improved preparation processes, which are easily carried out and lead to foundations with the mentioned stability characteristics.

### Summary

It has now been unexpectedly discovered that if foundation is prepared by mixing the necessary pigments not in their original state, but suitably separately pre-emulsified, it is possible to obtain foundation compositions with high chromatic stability. The invention relates to a method for the preparation of a foundation which involves mixing suitable quantities of precursor monochromatic emulsions, each of them containing a single pigment, in which said mixing gives the final foundation in the desired colour tone. The present new preparation method is particularly advantageous for the cosmetic industry and the product distribution: in particular, the availability of an adequate stock of the mentioned precursor emulsions ensures industry the potentiality of producing foundation batches in almost infinite colour tones: at the same time, only the required colour tones are actually selected and produced in real time, in connection with market requirements and their variations. Therefore, this production method allows optimising volumes and chromatic characteristics of the product in real time, avoids the creation and stocking of a material surplus and enables a better management of the orders and warehouse.

### Detailed description

According to the present invention, the final colour of the foundation is not determined by mixing of pure pigments (i.e. as such as they are available on the market, for example in powder form); in this invention, each pigment is first separately emulsified, thus obtaining a corresponding number of precursor emulsions: mixing the precursor emulsions in predetermined quantities leads to the obtainment of the foundation in the desired colour tone. Therefore, one of the characteristics of this invention lies in that each precursor emulsion is not simply monochromatic upon observation, but also mono-pigmented: its colour is not the result of different pre-mixed pigments, but of the use of one single pigment; consequently, every contact among pigments occurs exclusively by mixing emulsions separately containing them: said mixing leads to the desired final colour tone of the foundation.

In an advantageous embodiment of the invention, each precursor emulsion already has the complete composition of a normal foundation, differing from those known in that it contains one single pigment (red, yellow, black, etc.): in this case, the final foundation can be obtained directly by merely mixing the single precursor emulsions; these emulsions, having an identical composition, are perfectly miscible with one another and lead to foundations with high homogeneity, being quickly obtainable, with limited energy consumption, with no need of further ingredients and/or treatments.

The high miscibility of the precursor emulsions has very important implications: at the industrial level, for example, the preparation time was found to be substantially halved, compared to the traditional technique, as verified by tests carried out by the Applicant; in addition, the requirement of mixers with high energy consumption (turbo-mixers) is avoided, since traditional mixers can be used, quickly obtaining a product with high homogeneity. At the laboratory level, the high miscibility allows quickly obtaining new experimental colour tones in a reliable and reproducible manner.

In general, on any preparation scale, mixing these precursor mono-pigmented emulsions proved to be easier and immediate compared to the traditional mixing of basic ingredients (heterogeneous phases, such as powder pigments, lipophilic phases, hydrophilic phases, various additives, etc.). For the same reasons, it is extremely easy and immediate, both on an industrial and laboratory scale, to slightly adjust or alter the final colour by adding small portions of a mono-pigmented emulsion, whose formulation is equal to the one to be adjusted: these operations are much longer and more difficult if the adjustment occurs by adding pigments as such to the foundation, as known in the art. The finding that the production method is directly implementable on the common mixers used to produce the traditional foundations, with no need to adjust the plant, can be added to the aforesaid advantages.

The aforesaid preparation method proved to be advantageous also from the colour stability and reproducibility standpoint, enabling the obtainment of foundations high stability both within a single batch and among different batches. By stability within a batch, it is meant the colour constancy of foundation aliquots belonging to the same production batch, though obtained at different times. By stability among different batches, it is meant the colour constancy of foundations belonging to different batches, though obtained from the same original formulation.

The aforementioned effects are particularly amplified when the precursor emulsions are silicone-based emulsions (water in silicone) and the pigments used are of the types selected by the Applicant. Examples of silicone raw materials, useful for the preparation of the silicon-based emulsions are: Methicone and Dimethicone, Phenyl Trimethicone, Cyclopentasyloxane.

The pigments preferably selected by the Applicant are silicon-treated pigments, using silicones such as Methicone, Dimethicone or others. For information purposes, among the preferred pigments available on the market, those of the FDP series (Toshiki Pigment), AS series (ex. Wacker colouring agents), SA series (ex. Miyoshi Kasei), etc., can be mentioned.

Precursor emulsions are used in a limited number, preferably four; the colours of the single pigments contained therein, are preferably: black, red, yellow and white; the corresponding pigments are preferably: black iron oxide, red iron oxide, yellow iron oxide and titanium dioxide.

The pigment concentration within each emulsion and the quantities of emulsions to be mixed can widely vary depending on the final desired colour. Emulsions are prepared and combined with one another in such quantities so to obtain the qualitative-quantitative ratio of pigments necessary for achieving the desired target colour: the ratios between pigments in question, *per* se known in cosmetology, are herein advantageously applied to the corresponding mono-pigmented emulsions, obtaining the abovementioned benefits.

The remaining ingredients of the precursor emulsions are raw materials for cosmetic use, also commonly intended for the preparation of foundations. By way of a non-limiting example, we can mention: dispersants, moistening agents, emulsifying agents, suspending agents, preservatives, active ingredients, perfumes, etc.

Even the preparation methodology of the single emulsions can be chosen among those commonly known standards.

The fractioning of the foundation in the present precursor emulsions, allows also the preparation of the "on the spot" foundation. By "on the spot" preparation, we mean the preparation carried out only in case of real necessity: this preparation is, for example, that carried out on a personal scale by the end user, at the time of application on the skin; an "on the spot" preparation is also that made on the intermediate scale by the wholesaler or by the pharmacist, just before selling it; an "on the spot" preparation includes also the preparation carried out by the cosmetic industry itself, which can produce precursor emulsions as main finished product, reserving the production of final foundations only in targeted quantities and at the time of a real necessity, for example in response to a specific order. Such production method allows a significant modification of the whole production chain, making it substantially targeted to the obtainment / stocking / transport of a very limited number of precursor emulsions (preferably four), from which targeted productions of specific foundations are obtained. This methodology represents a basic difference compared to known productions, which aim from the start to obtain final foundations in innumerable colour tones: this methodology involved a complicate management of the warehouse and distribution, also exposing the manufacturer to the store of unsold foundations (typically in the least-used colours), or obliging him to separately produce and stock innumerable foundation colours, in different quantities depending on the estimated sale volume for each colour tone.

On the other hand, according to the invention, precursor emulsions are sold to the wholesaler, pharmacist or end user, while leaving these subjects the task of producing the final foundation at the desired moment; this preparation can preferably be carried out with the aid of special instructions (especially those for obtaining predetermined colour nuances) and possible means for easily performing the mixing.

Therefore, the invention includes also a kit for the preparation of a foundation including a specific number of mono-pigmented precursor emulsions, each emulsion preferably having the complete composition of a foundation and the following optional components: one or more containers to mix emulsions, one or more means to meter them, one or more means to mix them and instructions which indicate the quantities of emulsions to be used for each colour nuance.

### Example 1

By way of a non-limiting example of the present invention, four precursor emulsions were prepared with the same formulation base, made up of the following ingredients: water, cyclopentasiloxane, talc, mineral oil, glycerine, propylene glycol, Cetyl PEG/PPG-10/1, Dimethicone, Polyglyceril-3 diisostearate, sodium chloride, nylon 12, disteardimonium hectorite, dimethicone/vinyl dimethicone crosspolymer pigment, dimethicone/methicone copolymer pigment, tocopheryl acetate, sodium dehydroacetate, EDTA tetrasodium, phenoxyethanol, ethylhexylglycerin.

The only difference among the four emulsions consisted in the single pigment contained therein, that is titanium dioxide, yellow iron oxide, red iron oxide and black iron oxide, respectively. The precursor emulsions were then mixed with one another in such quantities as to obtain a final foundation emulsion containing:

| **Pigment** | **% Quantity (of the pigment total)** |
|---|---|
| | |
| Titanium dioxide | 44.7% |
| Yellow iron oxide | 21.5% |
| Red iron oxide | 19.7% |
| Black iron oxide | 14.1 % |

Mixing was carried out in a mixer of Melter/ Mixer type for pastes and cosmetic compounds, carried out at a speed of 45 rpm, for a total time of 20 minutes.

A silicone foundation of Beige No. 1 colour, with high homogeneity, was obtained. Repeated tests have highlighted a high colour stability and reproducibility, both within the same production batch and between different batches. In addition, the product has passed the following safety tests envisaged for placing cosmetic products on the market: stability test (stability of the formula over time), challenge test (effectiveness of the preservative system), patch test (skin irritation test).

## Claims

1. Method for the preparation of a foundation, comprising mixing of suitable quantities of precursor emulsions, each containing one single pigment, wherein said mixing gives the foundation in the desired colour tone.

2. Method according to claim 1, wherein precursor emulsions have the complete composition of a foundation, differing among them only regarding the pigment contained therein.

3. Method according to claims 1-2, wherein said emulsions are four in number, having respectively black, red, yellow and white colour.

4. Method according to claim 3, wherein the pigments used are: black iron oxide, red iron oxide, yellow iron oxide and titanium dioxide.

5. Method according to claims 1-4, where the pigments are silicone-treated pigments.

6. Method according to claims 1-5, wherein the precursor emulsions are water in silicone emulsions.

7. Method according to claims 1-6, carried out on industrial scale, where users receive the final product already mixed.

8. Method according to claims 1-6, individually carried out by the users, who choose the desired colour tone and mix precursor emulsions according to suitable proportion indications.

9. Kit for the preparation of a foundation including precursor: emulsions containing one single pigment and the remaining ingredients necessary to form a complete composition of a ready-to-use foundation, and one or more among the following components: containers to mix emulsions, means to meter them, means to mix them and instructions which indicate the quantity of emulsion to be used for each colour tone,

10. Kit according to claim 9, wherein the quantities of emulsions contained in the kit allow the preparation of one single foundation package for personal use.

11. Kit according to claim 9, wherein the quantities of emulsions contained in the kit allow the preparation of a quantity of foundation consistent with the requirements of a wholesaler, drugstore or another intermediate distributor.

## Patentansprüche

1. Verfahren zur Herstellung einer Grundierung, umfassend Mischen von geeigneten Mengen von Vorläuferemulsionen, die jeweils ein einzelnes Pigment enthalten, wobei das Mischen die Grundierung in dem gewünschten Farbton ergibt.

2. Verfahren gemäß Anspruch 1, wobei die Vorläuferemulsionen die vollständige Zusammensetzung einer Grundierung aufweisen, und sich untereinander nur bezüglich des darin enthaltenen Pigments unterscheiden.

3. Verfahren gemäß Ansprüchen 1-2, wobei die Emulsionen in der Anzahl von vier vorliegen, die jeweils eine schwarze, rote, gelbe, beziehungsweise weiße Farbe aufweisen.

4. Verfahren gemäß Anspruch 3, wobei die verwendeten Pigmente sind: schwarzes Eisenoxid, rotes Eisenoxid, gelbes Eisenoxid und Titandioxid.

5. Verfahren gemäß Ansprüchen 1-4, wobei die Pigmente silikon-behandelte Pigmente sind.

6. Verfahren gemäß Ansprüchen 1-5, wobei die Vorläuferemulsionen Wasser-in-Silikon-Emulsionen sind.

7. Verfahren gemäß Ansprüchen 1-6, in industriellem Maßstab ausgeführt, wobei Benutzer das Endprodukt bereits gemischt erhalten.

8. Verfahren gemäß Ansprüchen 1-6, individuell durch die Benutzer ausgeführt, die den gewünschten Farbton wählen und die Vorläuferemulsionen gemäß geeigneten Verhältnisangaben mischen.

9. Kit zur Herstellung einer Grundierung, umfassend Vorläuferemulsionen, die ein einzelnes Pigment und die übrigen notwendigen Inhaltsstoffe, um eine vollständige Zusammensetzung einer gebrauchsfertigen Grundierung zu bilden, enthalten, und einen oder mehrere unter den folgenden Bestandteilen: Behälter, um die Emulsionen zu mischen, Mittel, um sie zu messen, Mittel, um sie zu mischen, und Anleitungen, die die Menge an Emulsion angeben, die für jeden Farbton zu verwenden ist.

10. Kit gemäß Anspruch 9, wobei die Mengen an Emulsionen, die in dem Kit enthalten sind, die Herstellung von einer einzigen Grundierungspackung zum persönlichen Gebrauch erlauben.

11. Kit gemäß Anspruch 9, wobei die Mengen an Emulsionen, die in dem Kit enthalten sind, die Herstellung von einer Grundierungsmenge, die mit den Anforderungen eines Großhändlers, einer Drogerie oder eines anderen Zwischenhändlers übereinstimmen, erlauben.

## Revendications

1. Procédé pour la préparation d'un fond de teint, comprenant le mélange de quantités appropriées d'émulsions précurseurs, chacune contenant un unique pigment, ledit mélange donnant le fond de teint dans la couleur désirée.

2. Procédé selon la revendication 1, dans lequel les émulsions précurseurs ont la composition complète du fond de teint, ne s'en différenciant qu'en ce qui regarde le pigment contenu dedans.

3. Procédé selon les revendications 1 à 2, dans lequel lesdites émulsions sont au nombre de quatre, ayant respectivement la couleur noir, rouge, jaune et blanche.

4. Procédé selon la revendication 3, dans lequel les pigments utilisés sont : l'oxyde de fer noir, l'oxyde de fer rouge, l'oxyde de fer jaune et le dioxyde de titane.

5. Procédé selon les revendications 1 à 4, dans lequel les pigments sont des pigments traités à la silicone.

6. Procédé selon les revendications 1 à 5, dans lequel les émulsions précurseurs sont des émulsions eau dans silicone.

7. Procédé selon les revendications 1 à 6, mis en oeuvre à l'échelle industrielle, dans lequel les usagers reçoivent le produit fini déjà mélangé.

8. Procédé selon les revendications 1 à 6, mis en oeuvre individuellement par les usagers qui choisissent le ton de couleur souhaité et mélangent les émulsions précurseurs selon des indications de proportion appropriées.

9. Kit de préparation d'un fond de teint comprenant des émulsions précurseurs contenant un unique pigment et le reste des ingrédients nécessaires pour former une composition complète d'un fond de teint prêt à l'usage, et un ou plusieurs parmi les composants suivants : des récipients pour mélanger des émulsions, des moyens pour les mesurer, des moyens pour les mélanger et des instructions indiquant la quantité d'émulsion à utiliser pour chaque ton de couleur.

10. Kit selon la revendication 9, dans lequel les quantités d'émulsions contenues dans le kit permettent la préparation d'un unique lot de fond de teint pour un usage personnel.

11. Kit selon la revendication 9, dans lequel les quantités d'émulsions contenues dans le kit permettent la préparation d'une quantité de fond de teint compatible avec les exigences d'un grossiste, d'un drugstore ou de tout autre distributeur intermédiaire.
